# EUROPEAN PATENT APPLICATION

(11) **EP 0 753 513 A2**
(43) Date of publication of application: **15.01.1997**
(21) Application number: 96110980.8
(22) Date of filing: 08.07.1996
(51) Int. Cl.: C07D 249/08, C07F 7/10, A61K 31/41

(54) **Optically active triazole derivative, process for producing the same, antifungal agent, and use thereof**

(30) Priority: 08.07.1995 JP 196173/95
(71) Applicant: NIHON NOHYAKU CO., LTD., Chuo-ku, Tokyo (JP)
(72) Inventor: Kodama, Hiroki, Sakai-shi, Osaka (JP); Umimoto, Koji, Kawachinagano-shi, Osaka (JP); Kawaguchi, Michihiko, Tondabayashi, Osaka (JP); Shimosako, Masahiro, Nishiyoshino-mura, Yoshino-gun, Nara (JP); Yoshida, Masanori, Hashimoto-shi, Wakayama (JP)
(74) Representative: Hansen, Bernd, Dr. Dipl.-Chem.

(57) **Abstract**

An optically active triazole derivative represented by the following formula (I) is disclosed: 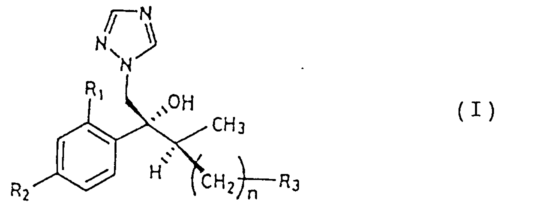 wherein R₁ and R₂ each represents a hydrogen atom, a halogen atom or a C₁-C₆ haloalkyl group; R₃ represents a phenyl group, a phenyl group substituted with a halogen atom, a C₁-C₆ haloalkyl group, a C₁-C₆ haloalkoxy group or a cyano group, or a silyl group substituted with a C₁-C₆ alkyl group, a phenyl group or a halogen-substituted phenyl group; and n is an integer of 1 or 2, provided that R₁ and R₂ are not hydrogen atoms at the same time, or a pharmaceutically acceptable salt thereof. A method for producing it, an antifungal agent comprising it, and the use thereof are also disclosed.

## Description

### FIELD OF THE INVENTION

The present invention relates to a novel optically active triazole derivative or a salt thereof, a process for producing it, an antifungal agent comprising the derivative or a pharmaceutical acceptable salt as an active ingredient, and the use thereof.

### BACKGROUND OF THE INVENTION

In recent years, fungus infection in immuno-compromised host which is caused by immunodeficiency or by the use of anticancer drugs has been a serious problem of inducing fungous diseases due to fungal infection. Thus, various antifungal agents have been developed to solve the problem.

EP-A-333059 discloses that triazole compounds having a structure similar to the derivative represented by formula (I) of the present invention described below have fungicidal activity for agricultural use; however, the medical use and activity against fungi infested in human or animals are not disclosed.

### SUMMARY OF THE INVENTION

An object of the present invention is to provide an optically active triazole derivative having an antifungal activity which is useful in treating human and animal mycosis.

This and other objects of the present invention have been attained by an optically active triazole derivative represented by the following formula (I): 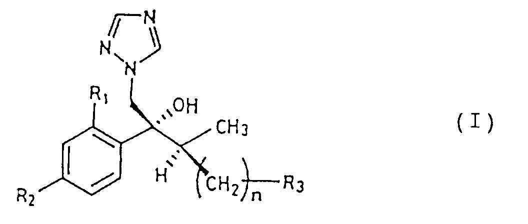 wherein R₁ and R₂ are the same or different and each represents a hydrogen atom, a halogen atom or a C₁-C₆ haloalkyl group; R₃ represents a phenyl group, a phenyl group having one or more substituents which are the same or different and are selected from the group consisting of a halogen atom, a C₁-C₆ haloalkyl group, a C₁-C₆ haloalkoxy group and a cyano group, or a silyl group having one or more substituents which are the same or different and are selected from the group consisting of a C₁-C₆ alkyl group, a phenyl group and a halogen-substituted phenyl group; and n is an integer of 1 or 2, provided that R₁ and R₂ are not hydrogen atoms at the same time, or a pharmaceutically acceptable salt thereof.

Furthermore, this and other objects of the present invention have been attained by a process for producing the optically active triazole derivative represented by formula (I), which comprises reacting a compound represented by the following formula (II): 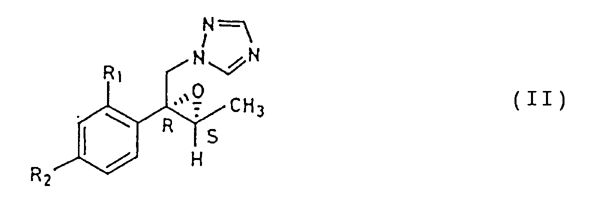 (wherein R₁ and R₂ are the same as those defined above) with a Grignard reagent represented by the following formula (III):

R₃-(CH₂)ₙMgx (III)

(wherein R₃ and n are the same as those defined above; and X represents a halogen atom).

Moreover, this and other objects of the present invention have been attained by a pharmaceutical composition which comprises as an active ingredient a pharmaceutically effective amount of the optically active triazole derivative represented by formula (I) or a pharmaceutically acceptable salt thereof, together with a pharmaceutically acceptable carrier or diluent.

Still furthermore, this and other objects of the present invention have been attained by a method for preventing or treating mycosis which comprises administering a pharmaceutically effective amount of the optically active triazole derivative represented by formula (I) or a pharmaceutically acceptable salt thereof, optionally together with a pharmaceutically acceptable carrier or diluent to human or animals in need of such prevention or treatment.

Still moreover, this and other objects of the present invention have been attained by use of the optically active triazole derivative represented by formula (I) or a pharmaceutically acceptable salt thereof for preparing an antifungal composition.

### DETAILED DESCRIPTION OF THE INVENTION

The racemic compounds represented by formula (I) have been reported (EP-A-0679647). The compounds containing two asymmetric carbon atoms have four stereo isomers. In EP-A-0679647, a mixture of four stereo isomers was divided into two paris of diastereomer: one is erythro and the other is threo. However, optical resolution of each diastereomer and asymmetric synthesis were not performed. Therefore, absolute structure necessary for mycotic activity is not known. The present invention has found the stereo structure of an optically active triazole derivative which has the most potent antifungal activity among these 4 isomers and salts thereof. Furthermore, a process for selectively producing the optically active triazole derivative has been also found.

In formula (I), examples of the halogen atom include chlorine, fluorine, bromine and iodine atoms. Examples of the C₁-C₆ alkyl group include linear or branched alkyl groups having from 1 to 6 carbon atoms such as methyl, ethyl, n-propyl, iso-propyl, n-butyl, iso-butyl, sec-butyl, tert-butyl, n-pentyl and n-hexyl groups. Examples of the C₁-C₆ haloalkyl group include haloalkyl groups having from 1 to 6 carbon atoms and substituted with one or more halogen atoms, which may be the same or different, such as chloromethyl, fluoromethyl, bromomethyl, iodomethyl, dichloromethyl, difluoromethyl, trifluoromethyl, chloroethyl, fluoroethyl, bromoethyl, iodoethyl, dichloroethyl, difluoroethyl, trifluoroethyl, chloropropyl, fluoropropyl and fluorobutyl groups. Examples of the C₁-C₆ haloalkoxy group include linear or branched haloalkoxy groups having from 1 to 6 carbon atoms and substituted with one or more halogen atoms, which may be the same or different, such as chloromethoxy, fluoromethoxy, trifluoromethoxy and trifluoroethoxy groups.

Preferably, R₁ and R₂ are the same or different and each represents a chlorine atom or a fluorine atom, and R₃ represents a phenyl group substituted with a chlorine atom, a fluorine atom or a cyano group.

The optically active triazole derivative of the present invention represented by formula (I) or an acid addition salt thereof may be used as an antifungal agent. Examples of the acid include inorganic acids (e.g., hydrochloric acid, sulfuric acid) and organic acids (e.g., oxalic acid, methanesulfonic acid, nitric acid).

The optically active triazole derivative represented by formula (I) can be produced by, for example, the following process. 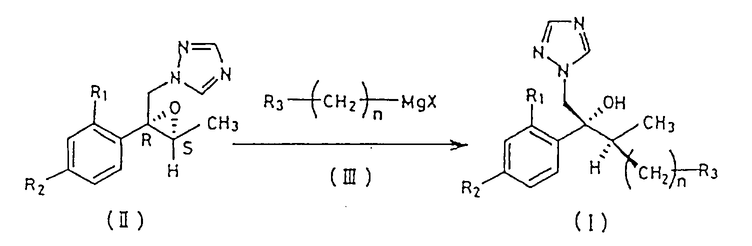 wherein R₁, R₂, R₃ and n are the same as those defined above; and X represents a halogen atom. In formula (III), examples of the halogen atom represented by X include chlorine, fluorine, bromine and iodine atoms.

The optically active triazole derivative represented by formula (I) can be produced by reacting a compound represented by formula (II), which can be produced by known methods (for example, those described in *Chem*. *Pharm*. *Bull*., 39:2241 (1991); *Chem Pharm*. *Bull*., 41:1035 (1993)) with a reactant prepared from a Grignard reagent represented by formula (III) and a copper salt. The compound represented by formula (II) was reported in EP-A-0421210 and EP-A-0473387 where the compound was used as starting material to synthesize antifungal compounds different from the structure in the present invention. The reaction of Grignard reagent and epoxide compounds in the prepense of copper salt was also shown in a few papers (*Org*. *Synth*., 69:1 (1990), *Tetrahedron*, 37:3879 (1981), *J*. *Org*. *Chem*., 48:4131 (1983)).

Although the copper salt may be used in a catalytic amount in general, it may be used in an appropriately selected amount ranging from the equivalent amount to the excessive amount. Examples of the copper salt include copper iodide, copper bromide, copper cyanide, and copper bromide/dimethylsulfoxide complex. If necessary, a Lewis acid, such as boron trifluoride, aluminum chloride, can be added.

The inert solvent for use in the reaction may be an arbitrary one, so long as the inert solvent does not inhibit the progress of the reaction. Examples thereof include inert solvents such as ether, tetrahydrofuran, n-hexane, toluene and a combination thereof.

Alternatively, the optically active triazole derivative of the present invention can be produced by optically resolving the racemic mixtures of the optically active triazole derivative represented by formula (I) by a conventional procedure such as optically active high performance liquid chromatography (HPLC) and preferential crystallization.

The optically active triazole derivative of the present invention represented by formula (I) is an antifungal agent useful for the prevention or treatment of human and animal mycosis such as topical fungal infection, mucosal fungal infection and systemic fungal infection with fungi (e.g., *Trichophyton*, *Candida*, *Aspergillus*).

When the derivative of the present invention is used as an antifungal agent, it is processed into dosage forms suitable for oral or parenteral administration (e.g., liquid, tablet, suppository, emulsion, ointment, cream, lotion, plaster) either alone or as a composition together with pharmaceutically acceptable inert carriers or diluents.

Although the amount administered can be any convenient amount according to conditions such as age, body weight, and administration mode, the antifungal agent of the present invention may be administered in a dose of from 0.05 to 100 mg/kg, preferably from 0.5 to 50 mg/kg, in one to several doses per day in the case of the systemic treatment of an adult.

In the case of topical treatment, the concentration of the active ingredient is preferably from 0.001 to 5%, and more preferably from 0.1 to 2%.

The antifungal agent of the present invention may be used together with other antifungal agents or antibacterial agents (e.g., amphotericin B, trichomycin, varitotin, clotrimazole.).

The present invention is now illustrated in greater detail by way of the following examples, but it should be understood that the present invention is not to be construed as being limited thereto.

### EXAMPLE 1

### Preparation of (2R,3S)-2-(2,4-difluorophenyl)-4-(4-fluorophenyl)-3-methyl-1-(1H-1,2,4-triazol-1-yl)butan-2-ol (Compound 1):

To a suspension of 200 mg of copper iodide in 8 ml of dry ether was added a solution of a Grignard reagent, which had been prepared from 145 mg of magnesium and 870 mg of 4-fluorobenzyl chloride, in 10 ml of dry ether at -30°C. Thirty minutes after stirring at -20°C, a solution of 500 mg of (2R,3S)-2-(2,4-difluorophenyl)-3-methyl-2-[(1H-1,2,4-triazol-1-yl)methyl]oxirane in 12 ml of dry ether was added thereto at the same temperature. Two hours after stirring at -20°C, a saturated aqueous solution of ammonium chloride was added and the object product was extracted with ethyl acetate. The organic layer was washed with a brine and dried over anhydrous magnesium sulfate. The solvent was distilled off under reduced pressure and the residue was purified by silica gel column chromatography (ethyl acetate : n-hexane = 2 : 1). Thus 466 mg of Compound 1 was obtained as a colorless oily product.

### EXAMPLE 2

### Preparation of (2R,3S)-2,4-bis(2,4-difluorophenyl)-3-methyl-1-(1H-1,2,4-triazol-1-yl)butan-2-ol (Compound 2):

To a suspension of 200 mg of copper iodide in 8 ml of dry ether was added a solution of a Grignard reagent, which had been prepared from 145 mg of magnesium and 1.24 g of 2,4-difluorobenzyl bromide, in 10 ml of dry ether at -30°C. Thirty minutes after stirring at -20°C, a solution of 500 mg of (2R,3S)-2-(2,4-difluorophenyl)-3-methyl-2-[(1H-1,2,4-triazol-1-yl)methyl]oxirane in 12 ml of dry ether was added thereto at the same temperature. Two hours after stirring at -20°C, a saturated aqueous solution of ammonium chloride was added and the object product was extracted with ethyl acetate. The organic layer was washed with a brine and dried over anhydrous magnesium sulfate. The solvent was distilled off under reduced pressure and the residue was purified by silica gel column chromatography (ethyl acetate : n-hexane = 2 : 1). Thus 603 mg of Compound 2 was obtained as a colorless oily product.

### EXAMPLE 3

### Preparation of (2R,3R)-2-(2,4-difluorophenyl)-3-methyl-4-trimethylsilyl-1-(1H-1,2,4-triazol-1-yl)butan-2-ol (Compound 5):

To a suspension of 200 mg of copper iodide in 8 ml of dry ether was added a 4.2 ml portion of a solution of a Grignard reagent, which had been prepared from 290 mg of magnesium and 1.46 g of chloromethyltrimethylsilane, in 12 ml of dry ether at -30°C. One hour after stirring at 10°C, a solution of 300 mg of (2R,3S)-2-(2,4-difluorophenyl)-3-methyl-2-[(1H-1,2,4-triazol-1-yl)methyl]oxirane in 10 ml of dry ether was added thereto at -20°C. Two hours after stirring at the same temperature, a saturated aqueous solution of ammonium chloride was added and the object product was extracted with ethyl acetate. The organic layer was washed with a saturated aqueous solution of sodium chloride and dried over anhydrous magnesium sulfate. The solvent was distilled off under reduced pressure and the residue was purified by silica gel column chromatography (ethyl acetate : n-hexane = 2 : 1). Thus 245 mg of Compound 5 was obtained as a colorless oily product.

### EXAMPLE 4

### Preparation of (2R,3S)-4-(4-cyanophenyl)-2-(2,4-difluorophenyl)-3-methyl-1-(1H-1,2,4-triazol-1-yl)butan-2-ol (Compound 7):

(1) In 150 ml of tetrahydrofuran, 19 g of 2,4-difluoropropiophenone was dissolved. Then 112 ml of 1 N solution of lithium hexamethyldisilazide in tetrahydrofuran was added dropwise thereinto over 20 minutes under cooling in a dry ice/acetone bath.
   After the completion of the addition, the mixture was stirred at the same temperature for 1 hour. A solution of 14 g of 4-cyanobenzyl bromide in 50 ml of tetrahydrofuran was added dropwise thereinto over 20 minutes and the resulting mixture was stirred at room temperature overnight. After adding a saturated aqueous solution of ammonium chloride thereto, the mixture was poured into water and extracted with ethyl acetate. The organic layer was washed with a brine and dried over anhydrous magnesium sulfate. The solvent was distilled off under reduced pressure and the residue was purified by silica gel column chromatography (ethyl acetate : n-hexane = 1 : 9). Thus 13 g of 2',4'-difluoro-2-methyl-3-(4-cyanophenyl)-2-propiophenone was obtained. Yield: 41%.
(2) In 50 ml of dimethyl sulfoxide, 1.0 g of 60% sodium hydride was suspended and stirred at 60°C for 1 hour. Then the reaction mixture was cooled below 15°C and 5.6 g of trimethylsulfoxonium iodide was added thereto. One hour after stirring at room temperature, the mixture was cooled below 15°C again and a solution of 2.4 g of 2,4-difluoro-2-methyl-3-(4-cyanophenyl)-propiophenone in 10 ml of dimethyl sulfoxide was added thereto. One hour after stirring at 60°C, the mixture was cooled to room temperature. Then it was poured into ice water and extracted with ethyl acetate. The organic layer was washed with a brine and dried over anhydrous magnesium sulfate. After distilling off the solvent under reduced pressure, 2.3 g of 2-(2,4-difluorophenyl)-3-methyl-(4-cyanophenyl)-1,2-butane oxide was obtained. Yield: 95%.
(3) In 50 ml of N,N-dimethylformamide, 2.3 g of 2-(2,4-difluorophenyl)-3-methyl-(4-cyanophenyl)-1,2-butane oxide was dissolved, and 2.1 g of 1,2,4-triazole and 2.0 g of potassium tert-butoxide were added thereto. Two hours after stirring at 100°C, the mixture was cooled to room temperature. Next, it was poured into ice water and extracted with ethyl acetate. The organic layer was washed with a brine and dried over anhydrous magnesium sulfate. After distilling off the solvent under reduced pressure, the residue was purified by silica gel column chromatography (ethyl acetate : n-hexane = 2 : 1) to give 1.5 g of 4-(4-cyanophenyl)-2-(2,4-difluorophenyl)-5-methyl-1-(1H-1,2,4-triazol-1-yl)butan-2-ol. Yield: 52%.
(4) In a solvent, 1.2 g of 4-(4-cyanophenyl)-2-(2,4-difluorophenyl)-5-methyl-1-(1H-1,2,4-triazol-yl-)butan-2-ol was dissolved and fractionated by using a fractional column (µ-Bondarsphere (Waters Co., LMT.); 19 mm × 15 cm) in high performance liquid chromatography. Thus 0.5 g of the erythro compound (shorter retention time) and 0.4 g of the threo compound (longer retention time) were obtained.

The erythro compound, i.e., (2R*,3S*)-4-(4-cyanophenyl)-2-(2,4-difluorophenyl)-3-methyl-1-(1H-1,2,4-triazol-1-yl)butan-2-ol was optically resolved using a fractional column in high performance liquid chromatography. Thus an optically active compound ([α]_{D} = - 47.3° (c = 0.11, methanol)) was obtained.

Table 1 shows typical examples of the optically active triazole derivatives represented by formula (I) which are prepared in accordance with the methods of the above Examples.

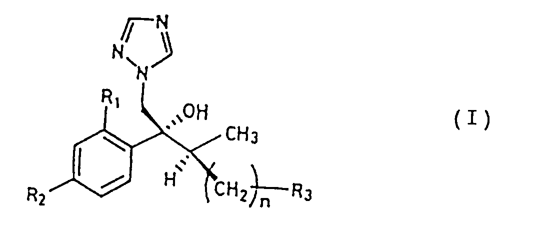

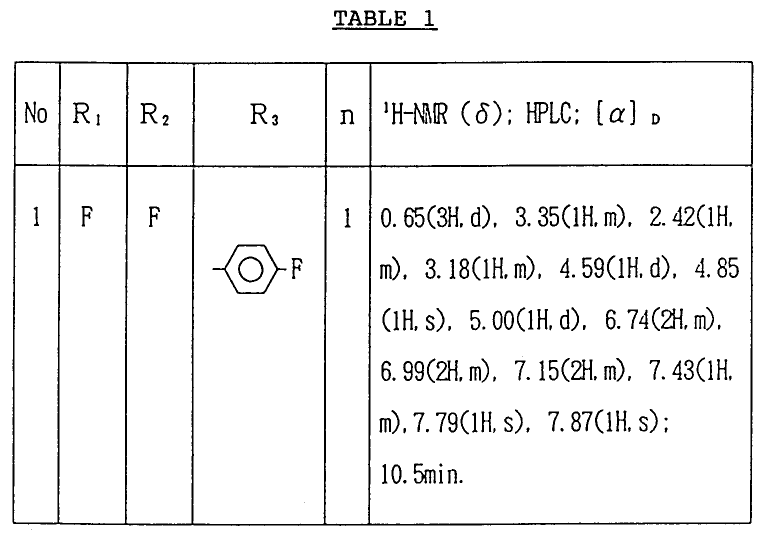

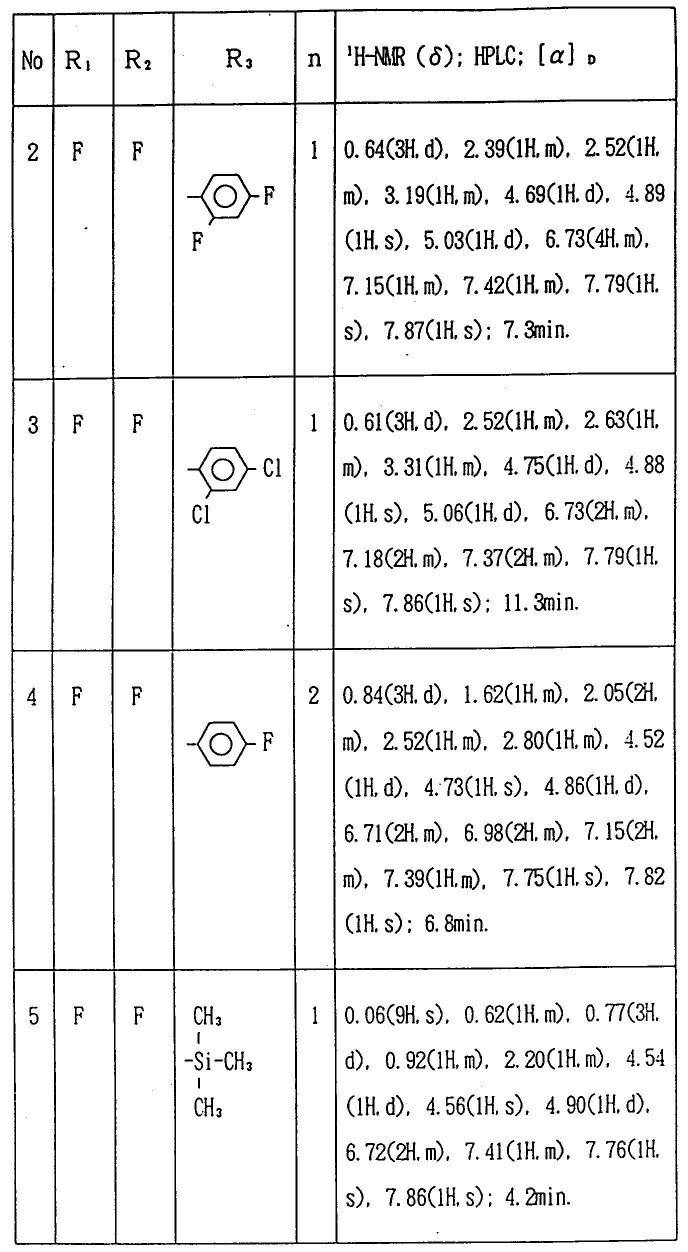

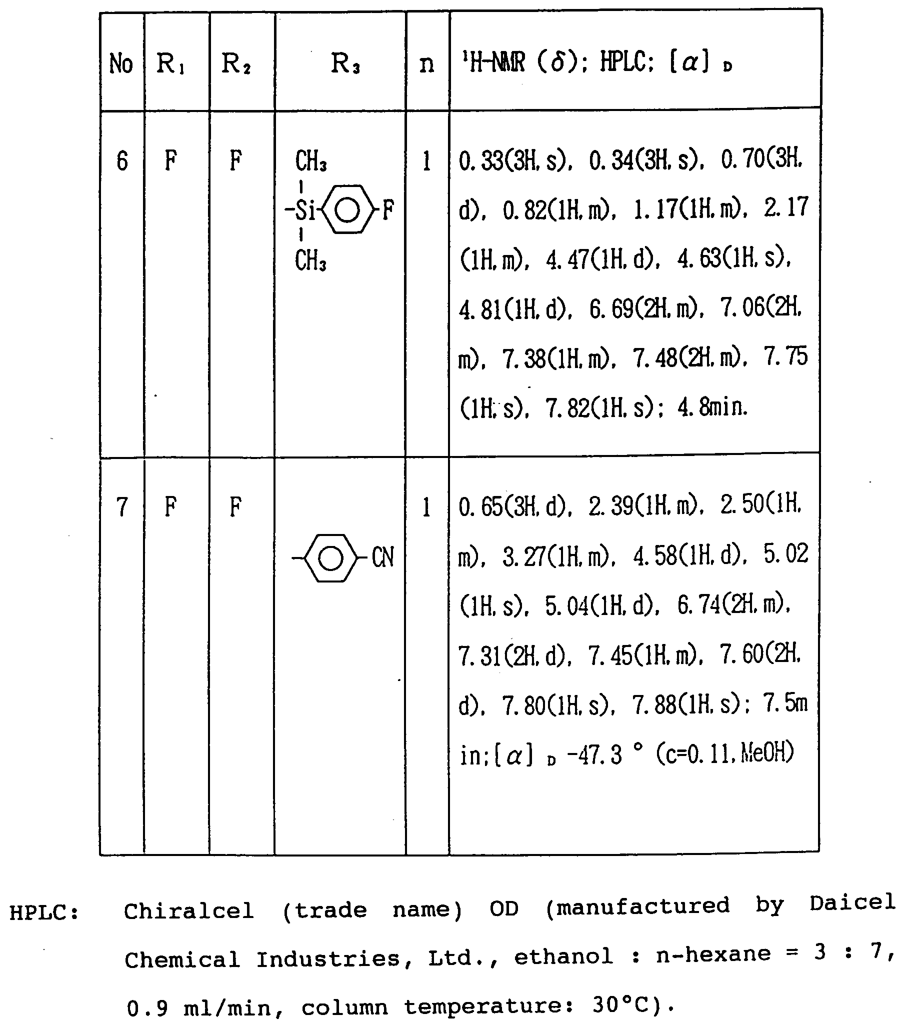

### FORMULATION EXAMPLE 1

| | |
|---|---|
| Invention compound | 0.01 part |
| 0.5% Carboxymethyl cellulose | 99.99 parts |

These components were suspended to give a suspension.

### FORMULATION EXAMPLE 2

| | |
|---|---|
| Invention compound | 1.00 part |
| Polyethylene glycol 400 | 99.00 parts |

These components were uniformly dissolved to give a liquid preparation for external application.

### FORMULATION EXAMPLE 3

| | |
|---|---|
| Invention compound | 2.00 parts |
| Polyethylene glycol 400 | 49.00 parts |
| Polyethylene glycol 4000 | 49.00 parts |

These components were molten under heating, mixed together and then cooled to give an ointment.

### FORMULATION EXAMPLE 4

| | |
|---|---|
| Invention compound | 3.00 parts |
| 1,2-Propanediol | 5.00 parts |
| Glycerol stearate | 5.00 parts |
| Whale wax | 5.00 parts |
| Isopropyl myristate | 10.00 parts |
| Polysorbate | 4.00 parts |

These components were heated and cooled. Then 68.00 parts of water were added thereto under stirring to give a cream.

### FORMULATION EXAMPLE 5

| | |
|---|---|
| Invention compound | 0.10 part |
| Stearyl alcohol | 5.00 parts |
| Cetanol | 5.00 parts |
| Medium-chain fatty acid triglyceride | 10.00 parts |
| Isopropyl myristate | 5.00 parts |
| Polysorbate 60 | 4.00 parts |
| Sorbitan monostearate | 1.00 part |
| Methyl paraoxybenzoate | 0.14 part |
| Propyl paraoxybenzoate | 0.06 part |
| Dibutyl hydroxytoluene | 0.02 part |
| Purified water | the balance |

A mixture of these components were treated in a conventional manner to give a cream.

### TEST EXAMPLE 1

Sabouraud's glucose broth (9.8 ml) was inoculated with 0.1 ml of *Candida albicans* IFO1270 (1.0×10⁶ cells/ml). Then 0.1 ml of a solution of each test compound dissolved with dimethylsulfoxide in an appropriate concentration was added to give concentrations of 10 µg/ml and 0.1 µg/ml. Two days after incubating at 37°C under shaking, the growth inhibition ratio was measured. Table 2 shows the results.

**TABLE 2**

| Compound | Growth inhibition ratio | | Remarks |
|---|---|---|---|
| | 10 µg/ml (%) | 0.1 µg/ml (%) | |
| 1 | 64 | 48 | Invention |
| 2 | 66 | 51 | " |
| 3 | 80 | 51 | " |
| 4 | 64 | 50 | " |
| 7 | 70 | 55 | " |
| A | 59 | 47 | Comparison |
| A: Cis-1-acetyl-4-[4-[[2-(2,4-dichlorophenyl)-2-(imidazol-1-ylmethyl)-1-3-oxolan-4- yl]methoxy]phenyl]piperazine (name: ketoconazole) | | | |

### TEST EXAMPLE 2

ddY mice aged 6 weeks were divided into a control group having 10 mice and test groups each having 5 mice. After inoculating with 4 ml/kg of *Candida albicans* IFO1270 (2.5×10⁷ cells/ml), which had been incubated on Sabouraud's glucose agar medium at 37°C for 2 hours, a 0.5% aqueous CMC solution (containing 10% of polyethylene glycol 400 and 0.1% of Tween 80) containing 0.02% of each test compound was orally administered to the mice in a dose of 5 ml/kg once a day for 4 days. The viable cells were counted for 10 days following the inoculation and the survival ratio on the final day was calculated. Table 3 shows the results.

**TABLE 3**

| Compound | Survival ratio (%) | Remarks |
|---|---|---|
| 1 | 80 | Invention |
| 2 | 60 | " |
| 3 | 100 | " |
| 4 | 40 | " |
| 7 | 100 | " |
| A | 40 | Comparison |

The same control drug in Example 1 was used as A.

The results show that the optically active triazole derivative of the present invention is an antifungal agent which is useful in treating mycosis.

While the invention has been described in detail and with reference to specific examples thereof, it will be apparent to one skilled in the art that various changes and modifications can be made therein without departing from the spirit and scope thereof.

## Claims

1. An optically active triazole derivative represented by the following formula (I): 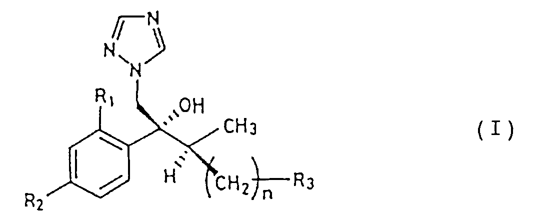 wherein R₁ and R₂ are the same or different and each represents a hydrogen atom, a halogen atom or a C₁-C₆ haloalkyl group; R₃ represents a phenyl group, a phenyl group having one or more substituents which are the same or different and are selected from the group consisting of a halogen atom, a C₁-C₆ haloalkyl group, a C₁-C₆ haloalkoxy group and a cyano group, or a silyl group having one or more substituents which are the same or different and are selected from the group consisting of a C₁-C₆ alkyl group, a phenyl group and a halogen-substituted phenyl group; and n is an integer of 1 or 2, provided that R₁ and R₂ are not hydrogen atoms at the same time, or a pharmaceutically acceptable salt thereof.

2. The optically active triazole derivative or the pharmaceutically acceptable salt thereof as claimed in claim 1, wherein R₁ and R₂ are the same or different and each represents a halogen atom; R₃ represents a phenyl group having one or more substituents which are the same or different and are selected from the group consisting of a halogen atom and a cyano group; and n is an integer of 1.

3. A process for producing an optically active triazole derivative represented by the following formula (I): 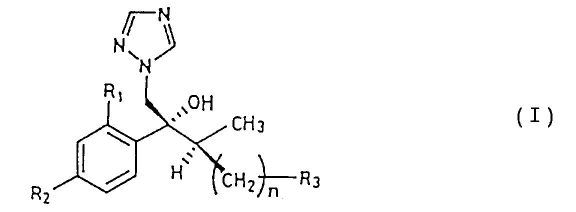 wherein R₁ and R₂ are the same or different and each represents a hydrogen atom, a halogen atom or a C₁-C₆ haloalkyl group; R₃ represents a phenyl group, a phenyl group having one or more substituents which are the same or different and are selected from the group consisting of a halogen atom, a C₁-C₆ haloalkyl group, a C₁-C₆ haloalkoxy group and a cyano group, or a silyl group having one or more substituents which are the same or different and are selected from the group consisting of a C₁-C₆ alkyl group, a phenyl group and a halogen-substituted phenyl group; and n is an integer of 1 or 2, provided that R₁ and R₂ are not hydrogen atoms at the same time,
which comprises reacting a compound represented by the following formula (II): 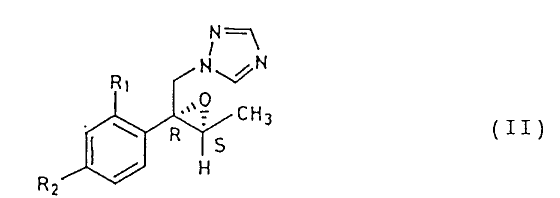 wherein R₁ and R₂ are the same as those defined above;
with a Grignard reagent represented by the following formula (III):
R₃-(CH₂)ₙMgX (III)
wherein R₃ and n are the same as those defined above; and X represents a halogen atom.

4. A pharmaceutical composition which comprises as an active ingredient a pharmaceutically effective amount of an optically active triazole derivative represented by the following formula (I): 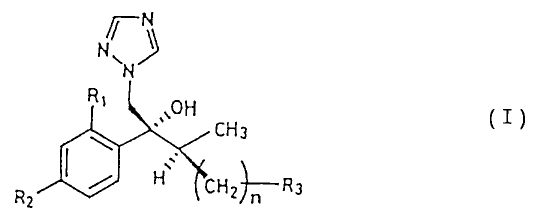 wherein R₁ and R₂ are the same or different and each represents a hydrogen atom, a halogen atom or a C₁-C₆ haloalkyl group; R₃ represents a phenyl group, a phenyl group having one or more substituents which are the same or different and are selected from the group consisting of a halogen atom, a C₁-C₆ haloalkyl group, a C₁-C₆ haloalkoxy group and a cyano group, or a silyl group having one or more substituents which are the same or different and are selected from the group consisting of a C₁-C₆ alkyl group, a phenyl group and a halogen-substituted phenyl group; and n is an integer of 1 or 2, provided that R₁ and R₂ are not hydrogen atoms at the same time, or a pharmaceutically acceptable salt thereof; together with a pharmaceutically acceptable carrier or diluent.

5. The pharmaceutical composition as claimed in claim 4, wherein R₁ and R₂ are the same or different and each represents a halogen atom; R₃ represents a phenyl group having one or more substituents which are the same or different and are selected from the group consisting of a halogen atom and a cyano group; and n is an integer of 1.

6. Use of an optically active triazole derivative represented by the following formula (I): 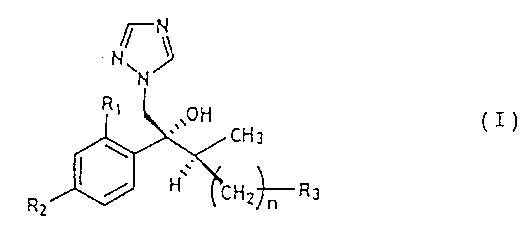 wherein R₁ and R₂ are the same or different and each represents a hydrogen atom, a halogen atom or a C₁-C₆ haloalkyl group; R₃ represents a phenyl group, a phenyl group having one or more substituents which are the same or different and are selected from the group consisting of a halogen atom, a C₁-C₆ haloalkyl group, a C₁-C₆ haloalkoxy group and a cyano group, or a silyl group having one or more substituents which are the same or different and are selected from the group consisting of a C₁-C₆ alkyl group, a phenyl group and a halogen-substituted phenyl group; and n is an integer of 1 or 2, provided that R₁ and R₂ are not hydrogen atoms at the same time, or a pharmaceutically acceptable salt thereof; optionally together with a pharmaceutically acceptable carrier or diluent , for preparing a medicament for preventing and/or treating fungal infections in humans or animals.

7. Use as claimed in claim 6 for preventing and/or treating mycosis.
